# EUROPEAN PATENT SPECIFICATION

(11) **EP 2 961 467 B1**
(45) Date of publication and mention of the grant of the patent: **17.07.2019**
(21) Application number: 14712823.5
(22) Date of filing: 27.02.2014
(51) Int. Cl.: A61M 31/00, A61J 7/00

(54) **BIOMATERIAL DELIVERY DEVICE**
BIOMATERIALABGABEVORRICHTUNG
DISPOSITIF D'ADMINISTRATION D'UN BIOMATÉRIAU

(30) Priority: 28.02.2013 US 201313781331
(43) Date of publication of application: 06.01.2016
(73) Proprietor: Medtronic Xomed, Inc., Jacksonville, FL 32216-0980 (US)
(72) Inventor: BERMAN, Phillip, J., Jacksonville, FL 32216-0980 (US); FRIEND, Matthew, J., Jacksonville, FL 32216-0980 (US); SHERMAN, Ethan, G., Jacksonville, FL 32216-0980 (US)
(74) Representative: Dehns
(86) International application number: PCT/US2014/019036
(87) International publication number: WO 2014/134312

(56) References cited:
- WO-A1-2008/138007
- US-A- 1 678 562
- US-A- 1 684 999
- US-A1- 2001 020 147

## Description

### RELATED APPLICATION

This application claims the benefit of U.S. Patent Application Serial No. 13/781,331 filed February 28, 2013, and entitled "BIOMATERIAL DELIVERY DEVICE".

### FIELD OF THE INVENTION

This invention relates to devices for delivering hygroscopic powdered biomaterials to surgical sites located in or near the respiratory tract.

### BACKGROUND

Adenoids (pharyngeal tonsils) and tonsils (palatine tonsils) are involved in a number of diseases of the ear, nose, and throat including chronic otitis media with effusion (COME), recurrent acute otitis media (RAOM), adenoiditis, pediatric chronic sinusitis, tonsillitis, pediatric obstructive sleep apnea (OSA), adult OSA, and chronic strep throat. Lingual tonsils can become infected and may cause or aggravate sore throat pain. Initial treatment for these various conditions normally involves administration of oral medications or, in the case of pediatric and adult sleep apnea, use of a continuous positive airway pressure (CPAP) device. Otitis media may be treated using ventilation tube surgery. Treatment success rates are often less than optimal, and in many cases the tonsils, adenoids or other throat tissue eventually may be surgically removed. Such surgeries are however painful, typically require the administration of anesthetics and lengthy post-operative recovery periods, and may be accompanied by complications such as post-operative bleeding, dehydration, weight loss, peritonsillar abscess, torticilis (neck stiffness), tissue regrowth, repeat surgery to address incomplete prior tissue removal, continued COME or RAOM, continued OSA, and occasionally death. Existing post-surgical treatments generally provide only limited relief, and may include dietary limitations, rinses, and administration of painkilling medications or oral antibiotics to reduce post-operative pain and infections.

U.S. Patent Application Publication No. US 2012/0108509 A1 describes an artificial scab composition for use in tonsillectomy, adenoidectomy and uvulopalatopharyngoplasty (UPPP) procedures. The composition is a substantially dry, free-flowing powdered mixture of at least partially solvatable chitosan particles and at least partially solvatable oxidized polysaccharide particles. When applied to a surgical site or wound moistened with bodily fluids, the powdered mixture forms an inhomogeneous, uncohesive, solid sheet-like body that breaks apart into smaller pieces if peeled away from the surgical site or wound. The powdered mixture may for example be applied using a bellows-type dispenser that expels the powder through an elongated, flexible straw that directs a stream of the powder onto a surgical site. U.S. Patent Nos. 1,678,562 and 1,684,999, U.S. Published Application No. 2001/0020147 A1 and International Application Publication No. WO 2008/138007 A1 disclose devices for the administration or delivery of powdered medicaments.

### SUMMARY OF THE INVENTION

If not aimed carefully or if excessive force is applied to the bellows, the above-described dispenser can inadvertently direct some or all of the powdered mixture into a human patient airway rather than on to the intended surgical site. Doing so is undesirable for a variety of reasons including potential patient safety risk and added delay in completing a surgical procedure.

Once the powder has been dispensed, it usually is desirable to spread the powder over the surgical site, for example, over the floor, walls and pillars of each tonsillar fascia, so as to form a thin film of the applied biomaterial. Doing so typically may require removal of the dispensing device from the patient's mouth and insertion of a spreading instrument, as there may not be sufficient room or viewing space to leave both the dispenser and spreading instrument in place while using one or the other. If upon spreading it appears that insufficient powder was applied, it may be necessary to remove the spreading instrument, reinsert the dispenser to apply more powder, and then to remove the dispenser and reinsert the spreading instrument to complete the procedure. Moreover, because it may be desirable to carry out spreading promptly after dispensing, a surgeon may prefer to complete all dispensing and spreading of powder on a first tonsillar fascia before dispensing and spreading powder on the remaining fascia. This too may necessitate repeated removal and insertion of the dispenser and spreading instrument.

It may also be desirable to retract nearby tissue while applying or spreading the powder. This may require insertion of an additional instrument into an already crowded operating field.

From the foregoing, it will be appreciated that what is needed in the art is a powdered biomaterial delivery device that can dispense and spread biomaterial powders in tonsillectomy, adenoidectomy, UPPP and other surgical procedures near patient airways. Such devices are disclosed and claimed herein.

The invention provides a biomaterial delivery device comprising an elongated handheld powder storage conduit sized for use in the mouth of a human patient and containing finely-divided powdered sterile biomaterial, the conduit having a proximal end, a distal end, and a bore having a central axis, the proximal end being closed by a movable powder dispensing actuator, the distal end (a) being closed by an openable sealing nib that can be moved from its closed to its open position by force upon the actuator and (b) having a distally-projecting powder discharge chute that spaces the nib away from a surgical site, wherein when the device is held over a surgical site with the proximal end uppermost and the nib actuated to its open position, powder passing the nib is deflected by the device at a first oblique direction with respect to the central axis and into or onto the powder discharge chute, and powder striking the chute is deflected by the device at a rotationally distinct second oblique direction with respect to the central axis and into or onto the surgical site.

Disclosed is also a method for applying a powdered biomaterial to a surgical site near a supine human patient airway, the method comprising the steps of:
a) holding in a generally upright position an elongated handheld powder storage conduit containing finely-divided powdered sterile biomaterial, the conduit having a proximal end, a distal end, and a bore having a central axis, the proximal end being closed by a movable powder dispensing actuator, the distal end (i) being closed by an openable sealing nib that can be moved from its closed to its open position by force upon the actuator and (ii) having a distally-projecting powder discharge chute that spaces the nib away from a surgical site, wherein when the device is held over a surgical site with the proximal end uppermost and the nib actuated to its open position, powder passing the nib is deflected by the device at a first oblique direction with respect to the central axis and into or onto the powder discharge chute, and powder striking the chute is deflected by the device at a rotationally distinct second oblique direction with respect to the central axis and into or onto the surgical site, and
b) applying force to the actuator so that powder is dispensed in non-aerosolized form past the nib and towards the surgical site.

The disclosed device and method enable rapid and accurate administration of powdered biomaterials onto or into surgical site near an airway of a supine patient. The disclosed nib desirably serves as a dump valve and powder deflector. The disclosed chute desirably serves as a further powder deflector that directs the falling powder onto a desired small target area. The chute desirably is formed in an inclined face of an angled tip having additional surfaces or contours that are usable for distributing or spreading the powder in or on a surgical site, and for retracting nearby tissue as need be. The disclosed device and method desirably permit single instrument powder dispensing, powder distribution and powder spreading. The disclosed device desirably is loosely filled with the powdered biomaterial, and the disclosed device and method desirably permit powder dispensing primarily via gravitational assistance without requiring the application of pressure on a cross-section of the powder itself (for example, from a piston, plunger, suction, compressed gas or other propellant) to force or sweep powder out of the device. This helps avoid powder clumping and clogging inside the delivery device and powder aerosolization or atomization at the delivery site, facilitates rapid and readily controllable operation by surgical personnel, and helps minimize inadvertent misdirection of biomaterial powder into the patient airway rather than into or onto the intended surgical site.

### BRIEF DESCRIPTION OF THE DRAWING

**Fig. 1** is an exploded view of one embodiment of the disclosed biomaterial dispensing device;
**Fig. 2** is a perspective view, partially in phantom, of the assembled **Fig. 1** device;
**Fig. 3** is a side orthogonal view of the assembled **Fig. 1** device, sectioned along line A-A' in **Fig. 4**;
**Fig. 5** is a side view of the assembled **Fig. 1** device;
**Fig. 6** is a perspective view of the **Fig. 1** nib;
**Fig. 7** is a perspective view of the distal end portion of the assembled **Fig. 1** device showing the nib in its closed position;
**Fig. 8** is a perspective view of the distal end portion of the assembled **Fig. 1** device showing the nib in its open position; and
**Fig. 9** through **Fig. 15** show various views of the disclosed tips.

Like reference symbols in the various figures of the drawing indicate like elements.

### DETAILED DESCRIPTION

The following detailed description describes certain embodiments and is not to be taken in a limiting sense. The terms shown below have the following meanings:

The term "airway" means a mammalian breathing passage, for example, as formed by the mouth, nose, throat and trachea.

The term "average inside diameter" when used in reference to a filled powdered delivery device having (a) a distal powder delivery outlet and (b) a powder-containing conduit having a central axis, a cylindrical or other cross-section and a constant or varying cross-sectional area along that axis means the diameter of a right circular cylinder having a height and volume like the height and volume circumscribed by the powder when the central axis of the filled device is aligned vertically with the outlet closed and directed downward.

The term "biomaterial" when used in reference to a substance means that the substance may be introduced into (and if need be left in) the body of a patient as part of a surgical procedure without significant deleterious or untoward effects upon the body.

The term "free-flowing" when used in reference to a powdered material means the powder will spontaneously flow downhill when placed on a horizontal surface and the surface rotated to an inclination of about 45° to 60° from the horizontal.

The term "gravitational assistance" when used in reference to a powdered substance dispensed from an outlet means that the substance passes through the outlet due to the influence of gravitational forces.

The term "gravitationally dispensed" when used in reference to a powdered substance dispensed from an outlet means that the substance passes through the outlet primarily due to gravitational assistance (for example, primarily by falling in a downward direction).

The term "hygroscopic" when used in reference to a powdered substance means that the substance takes up and retains water while in solid form (for example, by adsorption, absorption, or as chemically-bound water of hydration).

The term "loosely-filled" when used in reference to a powdered substance in a conduit or other container means that the substance is not packed into the container and will rearrange itself within the container if the container is slowly inverted without shaking.

The term "non-aerosolized" when used in reference to a powdered substance dispensed from an outlet means that the substance is not dispersed (for example, is not colloidally dispersed) into the surrounding atmosphere in free-floating form.

The term "non-expelled" when used in reference to a powdered substance dispensed from an outlet means that a piston or other component that applies force to the entire cross-sectional area of the dispensed powder portion is not used to dispense the powder in dense form.

The term "non-propelled" when used in reference to a powdered substance dispensed from an outlet means that a gaseous propellant is not used to accelerate the substance through the outlet or past the outlet (for example, via the Bernoulli effect).

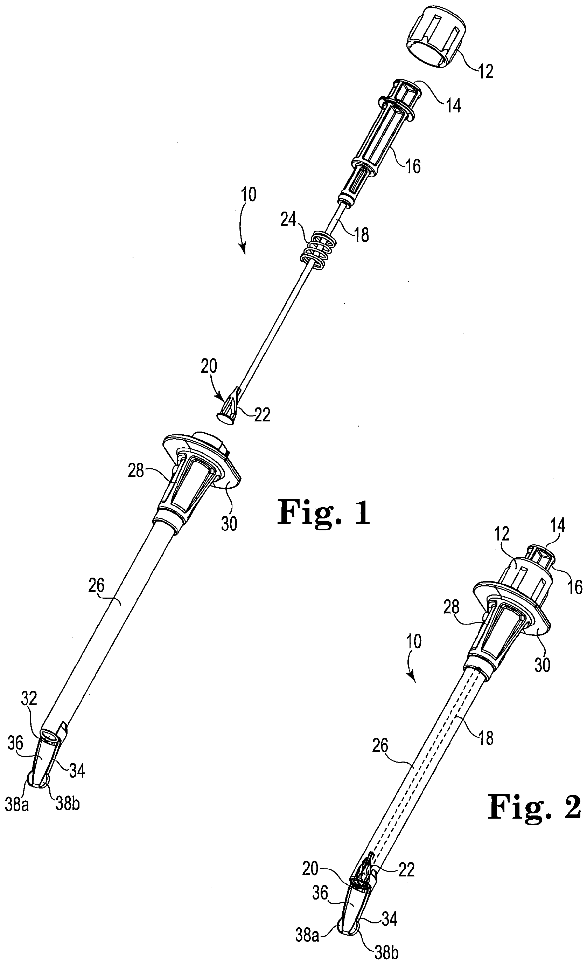

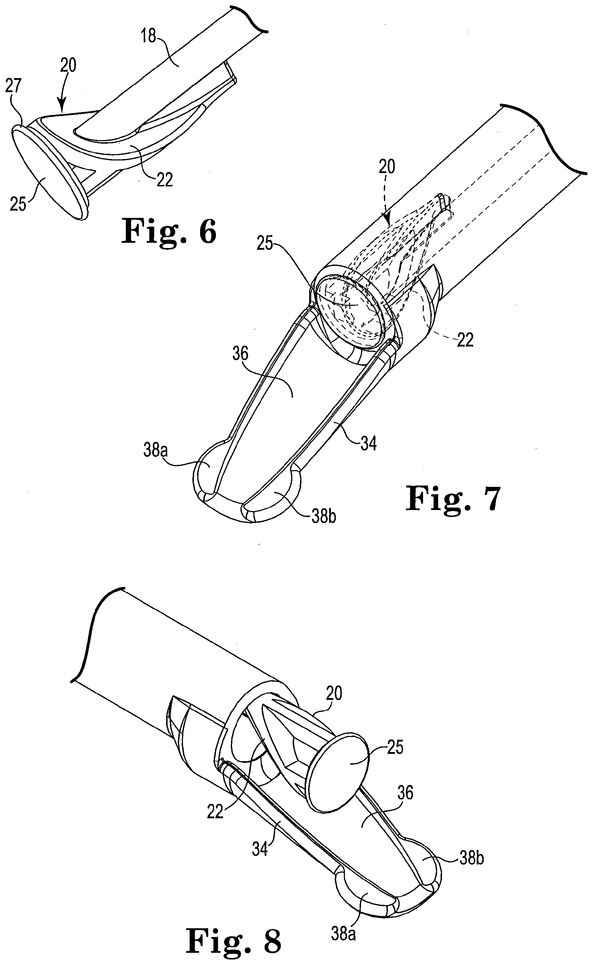

**Fig. 1** is a perspective exploded view depicting components that may be used in an embodiment **10** of the disclosed biomaterial delivery device. **Fig. 2** depicts a perspective view partly in phantom, of the assembled **Fig. 1** device. Retainer **12** is located at the proximal end of device **10** and provides a sleeve with openings sized to fit over and capture the end **14** of powder dispensing actuator **16**. In the embodiment shown in **Fig. 1** and **Fig. 2**, actuator **16** is integrally molded with an elongated and cylindrical, rod-like or stem-like member **18** that can be moved distally along the central axis of device **10** by depressing actuator **16** toward the distal end of device **10**. Actuator **16** is integrally molded with an elongated and cylindrical, rod-like or stem-like member **18** that can be moved distally along the device **10** central axis. Spring **24** biases actuator **16** toward the proximal end of device **10**. Nib **20** at the distal end of elongated member **18** moves from its closed to its open position when force is applied to actuator **16**, and in the embodiment shown in **Fig. 1** nib **20** returns from its open to its closed position when that force is released. Nib **20** desirably is repeatedly opened and closed by repeated application and release of such force. Nib **20** serves as a dump valve for a finely-divided powdered sterile biomaterial powder (not shown in **Fig. 1**) that will be contained in and dispensed by device **10**. Nib **20** includes a powder-deflecting ramp **22** that alters the trajectory of powder particles falling past ramp **22** by directing the particles at a first oblique direction with respect to the device **10** central axis. In the embodiment shown in **Fig. 1** and **Fig. 2** (and as may be seen more clearly in **Fig. 6**), ramp **22** is concave (for example, scoop-like) with respect to an axis perpendicular to the main powder flow direction so as to encourage the powder to remain in contact with ramp **22** as the powder is redirected and to discourage the powder from merely rebounding off ramp **22**. The disclosed ramp may instead have a generally flat face or may have other non-flat configurations, for example, concave (for example, trough-like) with respect to the main powder flow direction so as to direct the falling particles into a laterally more concentrated or narrower pattern as or after they leave ramp **22** or convex (for example, domed) with respect to the main powder flow direction so as to direct the falling particles into a laterally broadened pattern as or after they leave ramp **22**.

Member **18** fits loosely inside the bore of generally cylindrical or barrel-shaped conduit **26** so that powder may freely flow between member **18** and the inner sidewalls of conduit **26**. In the embodiment shown in **Fig. 1**, conduit **26** has a central cylindrical bore having a uniform inside diameter (and accordingly the same value for its average inside diameter) and a central axis, common with the central axis of elongated member **18**, along which the movable components of device **10** may reciprocally move towards and away from the distal end of device **10**. In the embodiment shown in **Fig. 1** and **Fig. 2**, the device central axis and the conduit or bore central axis are the same, and unless the context clearly indicates otherwise these terms may be used interchangeably. Actuator **16** desirably is sized to fit snugly but slidably inside a complementary cylindrical opening in grip **28**. Flange **30** on grip **28** assists a user of device **10** in holding, moving and actuating device **10** in one gloved hand, for example by holding device **10** with grip **28** placed between, and the distal surface of flange **30** in contact with, the user's gloved thumb and second finger or gloved first and second fingers, while meanwhile depressing actuator **16** using the free gloved index finger or free gloved thumb of the same hand. Meanwhile the user desirably may also tilt, rotate, press against, withdraw from or otherwise move device **10** as need be with respect to the surgical site.

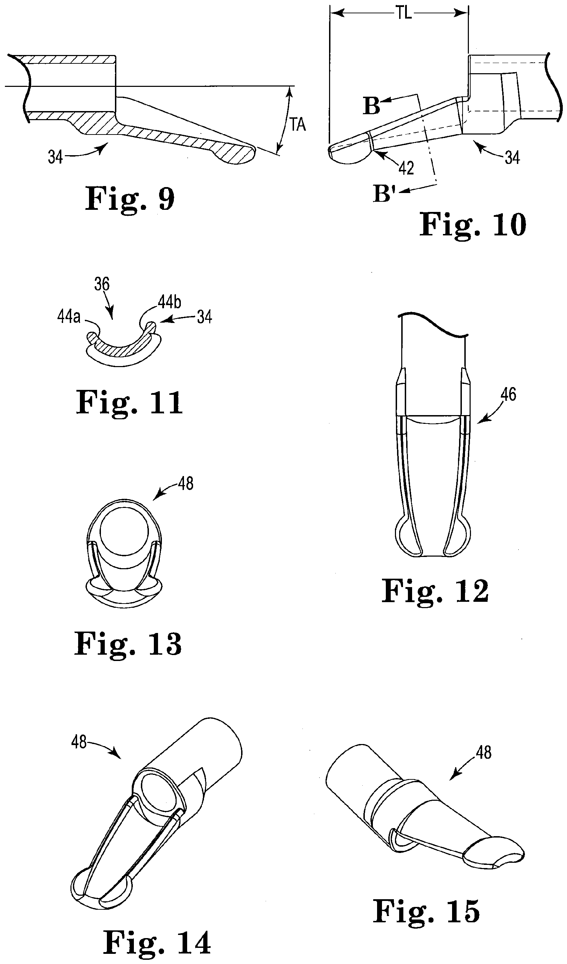

The distal outlet end **32** of device **10** is connected to a distally-projecting and desirably angled tip **34**. One face of tip **34** houses a distally-projecting powder-receiving and powder-delivering discharge chute **36** whose chute sidewalls (as may be better seen in **Fig. 14**) open toward the central axis of device **10**. Tip **34** spaces nib **20** away from a surgical site or other moist tissue surface, thereby helping to minimize moisture entry into device outlet **32** and discourage clogging. Chute **36** receives falling powder particles deflected by ramp **22**, and guides or otherwise deflects the received powder particles in a rotationally distinct second oblique direction (for example, with a rotational orientation with respect to the device **10** central axis that is 180° different than the above-mentioned first oblique direction). The thus-deflected powder particles are guided or otherwise directed by chute **36** into or onto a desired surgical site. In the embodiment shown in **Fig. 1**, the sidewalls of chute **36** converge as they approach the distal end of chute **36**, thereby helping to focus or concentrate the falling particles as or after they pass the distal end of chute **36**.

In the embodiment shown in **Fig. 1**, tip **34** also includes gently-rounded and flattened wing portions **38a** and **38b** which project laterally in hammerhead fashion away from the distal end of chute **36**. Wing portions **38a** and **38b** help in spreading and packing down the applied biomaterial, as well as widening and thereby reducing the pressure per unit area applied by the backside of tip **34** when tip **34** is pressed against a surgical site. Wing portions **38a** and **38b** and (as may be better seen in **Fig**. **5**) the gently-rounded backside of tip **34** are especially well adapted for manipulating (for example, retracting) tissue and for raking and spreading the dispensed powder across the floor and up and down the sidewalls of a surgical site, for example, a tonsillar fascia. The oblique angled orientation of tip **34** (*viz.*, the acute angle of tip **34** with respect to the device **10** central axis) further assists a user in retracting tissue, raking dispensed powder backwards across a tonsillar fascia floor, raking dispensed powder up the sidewalls of and into pockets or pillars in tonsillar fascia, and lining up the device outlet above the region at which dispensed powder is desired to land. The respective oblique angles of chute **36** and ramp **22** with respect to the device **10** central axis may each or both be chosen to help regulate the dispensed powder flow rate, with more oblique angles or more abrupt changes in direction generally causing reduced flow rates, and less oblique angles or less abrupt changes in direction generally causing increased flow rates. The gently-rounded contours and the absence of sharp edges on nib **20** and especially on tip **34** also help minimize surgical site trauma while device **10** is being used to dispense, distribute or spread powder.

Tip **34** may be firmly connected to the device, for example by being integrally molded with conduit **26**. If desired, tip **34** may instead be removable by a user and replaceable or interchangeable with other tips having different sizes (for example, pediatric and adult sizes) or different shapes or anatomically specific features that better adapt the tip to the intended surgical site, for example by providing specialized working surfaces that assist in depositing or spreading the biomaterial in complex or unusual surgical sites.

In the embodiment shown in **Fig. 1** and **Fig. 2**, elongated member **18** and the interior of conduit **26** each have a constant diameter. The tendency of some powders (for example, hygroscopic powders) to form clumps or cause clogging inside device **10** can be ameliorated by providing a plurality of powder-contacting clump-disrupting projections arrayed inside and along the length of the conduit between the actuator and the nib, the projections being movable in the direction of the device **10** central axis by force upon the actuator. Further details regarding such projections or regarding the presently-disclosed device may be found in copending US Patent No. 8,845,578.

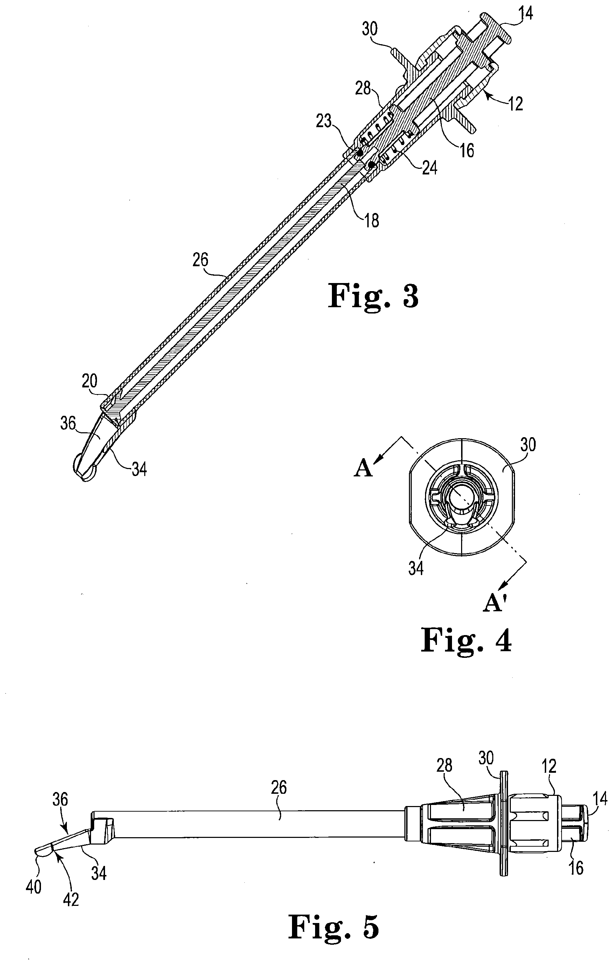

**Fig. 3** depicts is a side orthogonal view of the assembled **Fig. 1** device, sectioned along line A-A' in **Fig. 4**. The components shown in **Fig. 3** are like those in **Fig. 1** and **Fig. 2** but with the addition of a sealing O-ring **23**.

**Fig. 5** depicts a side view of the assembled **Fig. 1** device. Gently rounded dome **40** on the backside of tip **34** faces away from chute **36**. Dome **40** can serve among other things to distribute and thereby reduce the pressure per unit area applied by the edge of tip **34** when tip **34** is pressed against a surgical site. Dome **40** is especially well adapted for manipulating (for example, retracting) tissue and for raking and spreading the dispensed powder across the floor and up and down the sidewalls of a surgical site, for example, a tonsillar fascia. The small concave seam (see arrow **42**) where the proximal perimeter of dome **40** meets the backside of tip **34** provides an especially desirable improvement in powder raking and tissue retraction performance.

**Fig. 6** depicts a perspective view of nib **20**. Ramp **22** directs substantially all the falling powder away from the device **10** central axis. Ramp **22** does so by deflecting the falling powder at a first angled orientation with respect to the central axis and into or onto powder discharge chute **36** of tip **34** (not shown in **Fig. 6**). The end portion **25** of nib **20** also includes a discharge outlet sealing surface **27** that discourages powder from passing out of (and discourages powder, liquids and other materials from entering) the conduit when nib **20** is in its closed position.

**Fig. 7** and **Fig. 8** respectively depict perspective distal end portion views of the assembled **Fig. 1** device showing nib **20** in its closed (**Fig. 7**) and open (**Fig. 8**) positions. **Fig. 8** provides an especially useful illustration of the changes in powder direction that can arise when powder is deflected from ramp **22** to chute **36** while nib **20** is in the open position.

**Fig. 9** through **Fig. 15** provide several views of tips for use in the disclosed device. Most of the individual lead lines and numeric identifiers having been omitted for clarity. **Fig. 9** depicts a cross-sectional view of tip **34** through the device **10** central axis. The Angle **TA** may be referred to as the Tip Angle and may for example be about 4 to about 60, about 4 to about 50, about 6 to about 40 or about 8 to about 20 degrees with respect to the central axis. Steeper angles may make it easier to retract tissue, but may also make it more difficult to direct the dispensed powder onto a desired part of a surgical site.

**Fig. 10** depicts a side view of tip **34**. The length **TL** may be referred to as the Tip Length and may for example be about 10 to about 30, about 14 to about 25 or about 16 to about 20 mm measured from the device outlet to the distal end of tip **34**. TL values above about 12 mm are desirable in order to keep the nib dry during use, and values of about 12 to about 18-20 mm are desirable for use on pediatric and adult tonsillar fascia.

**Fig. 11** depicts a sectional view of tip **34**, sectioned along line B-B' in **Fig. 10**. Sidewalls **44a** and **44b** in trough **36** help guide the falling powder direct it toward the intended surgical site.

**Fig. 12** depicts the backside of tip **46**. Tip **46** is like tip **34** but lacks dome **40**. Tip **46** tends to have less effective spreading, raking and tissue retracting performance than a tip having the disclosed backside dome.

**Fig. 13**, **Fig. 14** and **Fig. 15** are three views of a removable and replaceable tip **48.** **Fig. 13** shows a view from the distal end toward the proximal end of tip **48**. **Fig. 14** and **Fig. 15** are respectively three quarter perspective views of the trough side (**Fig. 14**) and backside (**Fig. 15**) of tip **48**.

The disclosed biomaterial delivery device may be made in a variety of other embodiments. A number of design goals may be borne in mind when doing so. For example, the device is as mentioned sized for use in the mouth of a human patient, and desirably is sized for use above surgical sites located in the back of the throat of a supine human patient of adolescent or adult age. In general for such use there will be a desirable balance between the thinness of the device and the rapidity with which it can be used to dispense a needed quantity of biomaterial powder. Desirably the device when inserted through an open patient mouth and directed towards the tonsillar fascia is sufficiently thin so as to leave ample viewing room and space to insert and if need be to operate or manipulate other instruments or objects such as suction lines, lights, retractors (for example, Hurd retractors) or fingers (for example, for a hand other than the hand used to operate the disclosed device). The device desirably also is sufficiently long so that the dispenser when so inserted and directed will reach the back of the patient's throat. The device desirably has sufficiently great powder capacity and sufficiently rapid powder delivery capability to enable the desired powder amount to be dispensed, distributed and spread as need be in minimal time, for example, in less than two minutes, less than one minute or less than 30 seconds per tonsillar fascia for a tonsillectomy procedure. The device desirably provides a line of site view enabling a user to see simultaneously the chute, previously dispensed powder and the area on which powder will be dispensed with the next device actuation. The device desirably is shaped and sized to enable handheld, one-hand operation using either hand. The device may however be made in a variety of diameters and lengths, and may for example have a conduit with an outer diameter of about 4 to about 15 mm, an average inside diameter of about 5 to about 13 mm, and a length measured from the finger grip to the device outlet of about 10 to about 20 cm for use on tonsillar fascia and longer lengths (for example about 10 to about 25 cm) for use in nasal and sinus procedures. The disclosed flange desirably has a diameter of about 20 to about 40 mm, and may be equipped with flattened circumferential portions to keep the device from rolling when placed on a tray, table or other horizontal surface.

Desirably the device can be rotated at least 360° around its central axis while in the mouth, for example, in order to switch from right-handed to left-handed use, or to facilitate reorientation of the device such as when changing from one tonsillar fascia to the other. Accordingly, the device desirably does not include airlines or other potentially interfering appendages.

The device desirably can be used by itself to both dispense and spread metered amounts of powder and form a continuous powder coating over the entire surgical site. The device desirably is straight along the entire length of the conduit, as that can provide better visibility during use, and can provide better leverage and control when spreading powder using the tip.

The device desirably meters out an incremental powder portion each time the actuator is operated. The metered amount will be a function of several factors including the actuator stroke length and geometry, the chosen design for the elongated member and projections (if used), the chosen design for any seals employed, the device outlet size and shape, and the chosen nib design, tip design and associated angles. The device desirably permits dispensing to occur whether or not the device distal end is in contact with tissue or another surface, and desirably may be operated from its proximal end to dispense powder while the distal end is inserted in a patient's mouth without touching the back of the throat. The device also desirably permits dispensing to occur without having to shake the device. The device desirably is non-pressurized ("non-air-assisted"), desirably does not employ a propellant, suction (for example, inhaled air) or other moving gaseous stream that aerosolizes the dispensed powder, and desirably delivers most or all of the dispensed powder to the intended surgical site and none or substantially none of the dispensed powder to the surrounding tissue or the patient airway. The device accordingly and desirably would not be regarded as an inhaler and the dispensed powder desirably would be regarded as being non-aerosolized and non-propelled. The delivered biomaterial desirably falls down the conduit and through the device outlet primarily or exclusively due to gravitational forces. The delivered biomaterial desirably is not forced out of the device due to the action of a piston or other component that applies force to the entire cross-sectional area of the dispensed powder portion and dispenses such powder portion in dense form. The device accordingly and desirably would not be regarded as an injector and the dispensed powder desirably would be regarded as being non-expelled. The device desirably does not dispense powder or at least a significant quantity of powder if the device outlet is opened while the device centerline is horizontal, and desirably most readily dispenses powder when the device centerline is vertical or near-vertical. The device desirably keeps its powder dry until such time as the dispensed biomaterial contacts the surgical site. Desirably at least the portion of the device housing the stored biomaterial (for example, the conduit) and optionally also the elongated member, projections (if used) and tip are transparent or translucent. The use of transparent or translucent components can assist a surgeon in metering a desired powder amount during a procedure and at least initially in seeing where best to spread the powder.

The disclosed actuator desirably has a reciprocating actuation motion and more desirably a reciprocating motion along the device central axis. The motion desirably is in the distal direction, although an upward actuation in the proximal direction could be employed by using a suitable ledge or grip to facilitate lifting the actuator. The actuator may if desired use a rotating actuation motion but desirably does do so as such actuation may be more difficult to carry out while holding the device in a single gloved hand. For actuation using a reciprocating motion along the device central axis, actuation desirably requires a relatively short (for example, about 2 to about 12, about 4 to about 12 or about 6 to about 10 mm) stroke and a moderate but noticeable (for example, about 1 to about 4 Kg) force. Desirably the actuator (or if desired, one or more other components such as a projection or projections or the nib) are provided with one or more small ribbed or grooved portions that engage a nearby complementary recess or tang so as to provide a click stop or other tactile feedback actuation feature that informs the user (for example, during or at the end of an actuation stroke) that an incremental actuator movement has occurred. Such tactile feedback may also provide accompanying vibrations that can help agitate the powder. The disclosed actuator, elongated member, projections (if used) and nib desirably are connected to one another so that actuation causes all to move (for example, reciprocate) together. The actuator desirably is provided as part of a component integrally molded with at least the nib.

The disclosed nib desirably serves as both a closure valve for the device outlet and as a deflector that directs falling powder away from the device central axis and into a complementary chute structure in the disclosed tip. The disclosed device desirably is free from other powder-obstructing or powder-sealing valves located upstream from the nib. The nib may for example be connected to the actuator via the disclosed elongated member or may be opened and closed using some other connecting structure. Desirably the nib, elongated member and projections (if used) are formed (for example, molded) as a single integral component. The nib desirably discourages the powdered biomaterial from falling into areas where it is not intended to be placed and desirably also provides tactile feedback of dispenser actuation.

The disclosed conduit desirably has a bore (and most desirably a circular bore) whose diameter does not diminish along the length of the device from its proximal to distal ends. Reductions in bore diameter near the proximal end may not be a matter of concern, but reductions in bore diameter near the distal end and especially near the device outlet desirably are avoided. The device outlet desirably has the same cross-sectional shape as the bore (for example, is circular for a circular bore), desirably is as large as possible, and desirably has a diameter at least 75%, at least 80%, at least 90% or at least as large as the conduit average inside diameter.

The disclosed chute desirably directs the majority and desirably all of the falling powder onto a desired small target area that is displaced laterally from the device central axis. The chute diameter, measured as a chord between the tops of the chute sidewalls, desirably is about 5 to about 13 mm proximate the nib and about 3 to about 9 mm proximate the distal end of the chute. The tip desirably has a maximum width at its distal end that is at least about 120%, at least about 150%, at least about 200% or at least 300% as wide as the chute diameter at the chute distal end. The above-mentioned Tip Angle desirably is less than 90° with respect to the device central axis. The direction at which dispensed powder exits the device desirably does not change with successive operations (for example, strokes) of the device actuator. The device and its tip desirably lack needles, sharp edges or other like projections that could cause tissue penetration or other tissue trauma, and desirably includes wings or other powder spreading appendages with rounded distal edges. The tip desirably is sufficiently stiff to permit spreading and tamping the dispensed powder without noticeable tip deflection. The tip may however be bendable or otherwise formable to accommodate particular anatomical features or surgical sites.

The disclosed device and its non-powdered components may be made from a variety of materials including acrylics, olefins such as polyethylene and polypropylene, polycarbonates and other suitable thermoplastic or thermoset materials. The chosen materials desirably have a low tendency to adhere to the chosen biomaterial powder whether due to static or other attractive forces. The device may also include antistatic surface treatments, antistatic additives or surface finishes chosen so as to further discourage powder adhesion.

A wide variety of powdered biomaterials may be packaged in the disclosed device. The biomaterials desirably are organic but for some surgical end uses may be inorganic materials (for example, calcium carbonates, zeolites and other minerals or salts). Exemplary biomaterials may for example be natural materials (for example, polysaccharides), chemically modified natural materials (e.g, polysaccharide reaction products including oxidized, acetylated, deacetylated, acid-reacted, anhydride-reacted, esterified, neutralized, base-reacted, vinyl-group reacted, isocyanate-reacted or otherwise functionalized polysaccharides, polysaccharide salts and other polysaccharide derivatives), or synthetic materials (for example, polyglycolides, polyactides, poly(lactide-co-glycolides), poly(ε-caprolactones), poly(dioxanones), polyanhydrides, polyorthoesters, polyethylene glycols and polyvinyl alcohols). The powder may be a single material or mixture of materials (for example, a mixture of a crosslinkable material and a crosslinker). Exemplary biomaterials include agars, alginates, carrageenans, celluloses, chitins, chitosan, chondroitin sulfates, dextrans, galactomannans, glycogens, hyaluronic acids, starches, oxidized cellulose, oxidized chitin, oxidized chitosan, oxidized chondroitin sulfate, oxidized dextran, oxidized glycogen, oxidized hyaluronic acid, oxidized starch and other materials that will be known to persons having ordinary skill in the art. Suitable biomaterials may be obtained from a variety of commercial sources including CarboMer Inc., Monomer-Polymer and Dajac Labs, Inc. and Sigma-Aldrich Co. The powdered biomaterial desirably is in dry particulate form, for example, as free-flowing granules whose average particle diameter is less than about 1 mm, less than about 100 µm, about 1 to about 80 µm, or less than 1 µm, and accordingly the biomaterial desirably is not a compressed cake, paste, moistened clump-prone mass or other non-free-flowing material. The powdered biomaterial may be comminuted, lyophilized, crystallized or recrystallized if desired. If a mixture of particles is employed, the particles desirably are intimately mixed together prior to placement in the device, and further mixing desirably is not required at the point of use. The biomaterial may provide a variety of features such as the formation of a protective, mucoadhesive, biodegradable, bioresorbable, drug eluting or hemostatic structure (for example, a layer) following application to a surgical site. The biomaterial desirably is substantially collagen-free and more desirably contains no collagen at all so as to be saleable worldwide for use without restriction in humans. The biomaterial may optionally include a variety of other ingredients that are themselves dry, or which when mixed with the biomaterial will provide or can be processed (for example, dried) to provide a dry powdered biomaterial. Exemplary such other ingredients include acids, antifoam agents, antimicrobial agents, antioxidants, antistatic agents, bases, buffering agents, colorants, flow aids, hyperosmolar agents, indicators, flavoring agents, sweetening agents, therapeutic agents, modifiers to alter the release rate of therapeutic agents, and other adjuvants that will be familiar to persons having ordinary skill in the art. For example, a useful list of therapeutic agents may be found in U.S. Patent Application Publication No. US 2007/0264310 A1. The biomaterials desirably do not contain ingredients which might potentially harm mucosal tissues or structures. The disclosed device is especially desirable for dispensing hygroscopic powders that are prone to clumping or sticking. The disclosed device desirably contains the total volume of material to be used in an intended surgical procedure, for example about 0.5 to 2 grams per device for tonsillectomy procedures.

The disclosed devices desirably are ready-to use disposable items designed for one-time use. Following filling with the desired biomaterial powder, the device typically will be placed in suitable sealed packaging (for example, a metalized foil pouch and optional box) and subjected to sterilization prior to shipment to an end user. Exemplary sterilization techniques will be familiar to persons having ordinary skill in the art, and include gamma radiation, electron beam (E-Beam) processing, and cold ionizing radiation sterilization (for example, cold E-Beam sterilization) as described in published PCT Application No. WO 2009/132229 A2. The end user may thereafter remove the sterilized device from its sealed packaging in a suitably disinfected setting (for example, an operating room or other surgical location) and dispense the sterilized biomaterial onto the desired surgical site.

The disclosed device typically will be used by a surgeon near the conclusion of a surgical procedure. For example, a tonsillectomy or adenoidectomy may be carried out using traditional steps, with tissue excavation being performed using electrocauterization, snares, scalpels or other techniques, followed promptly thereafter by use of the device to dispense and desirably also to distribute or spread a coating of the disclosed powdered biomaterial on the exposed fascia. The application technique is not unlike frosting a cake using the backside of a spoon, but may be carried out much more quickly. Some surgeons may prefer to apply several metered powder doses and then spread the powder, and others may prefer repeatedly to apply a powder dose, spread the powder and repeat until a desired degree of coating is obtained. Desirably, substantially identical powder amounts are dispensed with each actuation stroke. As a general guide for tonsillectomy or adenoidectomy procedures, a recommended application rate and dose may be about 10-25 actuations per tonsil, dispensing about 0.02 to 0.05 g per actuation stroke.

Although specific and in some cases preferred embodiments have been illustrated and described, it will be appreciated by those of ordinary skill in the art that a wide variety of alternate or equivalent embodiments calculated to achieve the same purposes may be substituted for the specific embodiments shown and described without departing from the present invention. This application is intended to cover any adaptations or variations of the preferred embodiments discussed herein. Therefore, it is manifestly intended that this invention be limited only by the claims and the equivalents thereof.

## Claims

1. A biomaterial delivery device (10) comprising an elongated handheld powder storage conduit (26) sized for use in the mouth of a human patient and containing finely-divided powdered sterile biomaterial, the conduit having a proximal end, a distal end, and a bore having a central axis, the proximal end being closed by a movable powder dispensing actuator (16), the distal end (a) being closed by an openable sealing nib (20) that can be moved from its closed to its open position by force upon the actuator and (b) having a distally-projecting powder discharge chute (36) that spaces the nib away from a surgical site; **characterised in that** when the device is held over a surgical site with the proximal end uppermost and the nib actuated to its open position, powder passing the nib is deflected by the device at a first oblique direction with respect to the central axis and into or onto the powder discharge chute, and powder striking the chute is deflected by the device at a rotationally distinct second oblique direction with respect to the central axis and into or onto the surgical site.

2. A device according to claim 1 wherein the powder comprises a free-flowing and hygroscopic polysaccharide, polysaccharide reaction product or polysaccharide derivative.

3. A device according to claim 1 wherein the actuator has a reciprocating actuation motion along the central axis.

4. A device according to claim 1 wherein the actuator dispenses 0.02 to 0.05 g powder per actuation.

5. A device according to claim 1 wherein the nib comprises a powder-deflecting ramp (22) that directs substantially all passing powder away from the central axis.

6. A device according to claim 5 wherein the nib ramp is concave with respect to an axis perpendicular to the main powder flow direction so as to encourage passing powder to remain in contact with the ramp as the powder is deflected and discourage passing powder from rebounding off the ramp.

7. A device according to claim 1 wherein the chute has a distal end, and sidewalls that converge as they approach the chute distal end, thereby helping to concentrate the passing powder.

8. A device according to claim 7 wherein the chute has a diameter, measured as a chord between the tops of the chute sidewalls, of 5 to 13 mm proximate the nib and 3 to 9 mm proximate the chute distal end.

9. A device according to claim 1 further comprising a tip (34) having gently-rounded and flattened wing portions (38a, 38b) which project laterally away from the distal end of the chute and assist in powder spreading.

10. A device according to claim 9 wherein the tip lacks sharp edges that might cause tissue trauma.

11. A device according to claim 9 wherein the tip has a backside domed portion that assists in retracting tissue and in raking and spreading dispensed powder across the floor and up and down the sidewalls of a surgical site.

12. A device according to claim 9 wherein the tip is sufficiently stiff to permit spreading and tamping the dispensed powder without noticeable tip deflection.

13. A device according to claim 9 wherein the tip is removable by a user from the device and replaceable or interchangeable with tips (46, 48) having different sizes, different shapes or anatomically specific features that better adapt the tip to a surgical site.

14. A device according to claim 9 wherein the tip extends at an angle of 4 to 60 degrees with respect to the central axis and has a length of 10 to 30 mm.

15. A device according to claim 1 wherein the device is in sealed sterilized packaging.

## Patentansprüche

1. Biomaterialabgabevorrichtung (10), umfassend eine längliche, handgeführte Pulvervorratsleitung (26), die zur Verwendung im Mund eines menschlichen Patienten bemessen ist und feinzerteiltes pulverisiertes steriles Biomaterial enthält, wobei die Leitung ein proximales Ende, ein distales Ende und eine Bohrung aufweist, die eine Mittelachse aufweist, wobei das proximale Ende durch eine bewegliche Pulverabgabebetätigungsvorrichtung (16) verschlossen ist, das distale Ende (a) durch eine zu öffnende Dichtungsnase (20) verschlossen ist, die von ihrer geschlossenen in ihre geöffnete Position durch Kraft auf die Betätigungsvorrichtung bewegt werden kann, und (b) eine distal vorstehende Pulverausgaberutsche (36) aufweist, die die Nase von einer Operationsstelle beabstandet; **dadurch gekennzeichnet, dass**, wenn die Vorrichtung über eine Operationsstelle mit dem proximalen Ende zuoberst und der Nase in ihrer geöffneten Position betätigt, gehalten wird, das Pulver, das durch die Nase läuft, von der Vorrichtung in einer ersten schrägen Richtung in Bezug auf die Mittelachse und in oder auf die Pulverausgaberutsche abgelenkt wird und das Pulver, das auf die Rutsche auftrifft, durch die Vorrichtung in einer rotatorisch unterschiedlichen zweiten schrägen Richtung in Bezug auf die Mittelachse und in oder auf die Operationsstelle abgelenkt wird.

2. Vorrichtung nach Anspruch 1, wobei das Pulver ein rieselfähiges und hygroskopisches Polysaccharid, Polysaccharid-Reaktionsprodukt oder Polysaccharid-Derivat umfasst.

3. Vorrichtung nach Anspruch 1, wobei die Betätigungsvorrichtung eine hin- und hergehende Betätigungsbewegung entlang der Mittelachse aufweist.

4. Vorrichtung nach Anspruch 1, wobei die Betätigungsvorrichtung 0,02 bis 0,05 g Pulver je Betätigung abgibt.

5. Vorrichtung nach Anspruch 1, wobei die Nase eine Pulverablenkrampe (22) umfasst, die im Wesentlichen das gesamte durchlaufende Pulver von der Mittelachse weg leitet.

6. Vorrichtung nach Anspruch 5, wobei die Nasenrampe konkav in Bezug auf eine Achse ist, die senkrecht zur Hauptpulverfließrichtung verläuft, so dass das durchlaufende Pulver stärker in Kontakt mit der Rampe gehalten wird, wenn das Pulver abgelenkt wird, und das durchlaufende Pulver davon abgehalten wird, von der Rampe wegzuspringen.

7. Vorrichtung nach Anspruch 1, wobei die Rutsche ein distales Ende und Seitenwände aufweist, die in Richtung zum distalen Rutschenende hin zusammenlaufen, was dazu beiträgt, das durchlaufende Pulver zu sammeln.

8. Vorrichtung nach Anspruch 7, wobei die Rutsche einen Durchmesser, gemessen als Sehne zwischen den Oberseiten der Rutschenseitenwände, von 5 bis 13 mm nahe der Nase und 3 bis 9 mm nahe dem distalen Rutschenende aufweist.

9. Vorrichtung nach Anspruch 1, ferner umfassend eine Spitze (34), die sanft abgerundete und abgeflachte Flügelabschnitte (38a, 38b) aufweist, die seitlich von dem distalen Ende der Rutsche weg hervorragen und die Pulververteilung unterstützen.

10. Vorrichtung nach Anspruch 9, wobei die Spitze keine scharfen Kanten aufweist, die eine Gewebeverletzung verursachen könnten.

11. Vorrichtung nach Anspruch 9, wobei die Spitze einen an der Hinterseite gewölbten Abschnitt umfasst, der das Zurückziehen von Gewebe und das Verstreichen und Verteilen des abgegebenen Pulvers über dem Boden und die Seitenwände einer Operationsstelle nach oben und unten unterstützt.

12. Vorrichtung nach Anspruch 9, wobei die Spitze ausreichend starr ist, um das Verteilen und Stampfen des abgegebenen Pulvers ohne merkliche Spitzenablenkung zu ermöglichen.

13. Vorrichtung nach Anspruch 9, wobei die Spitze durch einen Benutzer von der Vorrichtung entfernt werden kann und durch Spitzen (46, 48) ersetzbar ist, die verschiedene Größen, verschiedene Formen und anatomisch spezifische Merkmale aufweisen, die die Spitze besser an eine Operationsstelle anpassen, oder gegen solche austauschbar ist.

14. Vorrichtung nach Anspruch 9, wobei die Spitze sich in einem Winkel von 4 bis 60 Grad in Bezug auf die Mittelachse erstreckt und eine Länge von 10 bis 30 mm aufweist.

15. Vorrichtung nach Anspruch 1, wobei sich die Vorrichtung in einer versiegelten sterilisierten Verpackung befindet.

## Revendications

1. Dispositif d'administration d'un biomatériau (10) comprenant un conduit de stockage de poudre portable allongé (26) dimensionné pour une utilisation dans la bouche d'un patient humain et contenant un biomatériau stérile en poudre à fines particules, le conduit ayant une extrémité proximale, une extrémité distale et un alésage doté d'un axe central, l'extrémité proximale étant fermée par un actionneur de distribution de poudre mobile (16), l'extrémité distale (a) étant fermée par une pointe d'obturation ouvrable (20) mobile et pouvant passer d'une position qui peut passer de sa position fermée à sa position ouverte sous l'effet d'une force exercée sur l'actionneur (b) et comportant une goulotte d'administration de poudre à projection distale (36) qui sépare la pointe d'un champ opératoire, **caractérisé en ce que** lorsque le dispositif est maintenu au-dessus d'un champ opératoire avec l'extrémité proximale en haut et la pointe actionnée dans sa position ouverte, la poudre passant par la pointe est déviée par le dispositif dans une première direction oblique par rapport à l'axe central et dans ou sur la goulotte d'administration de poudre, et la poudre venant frapper la goulotte est déviée par le dispositif dans une seconde direction oblique distincte du point de vue rotatif par rapport à l'axe central et dans ou sur le champ opératoire.

2. Dispositif selon la revendication 1 dans lequel la poudre comprend un polysaccharide à écoulement libre et hygroscopique, un produit réactionnel de polysaccharide ou un dérivé de polysaccharide.

3. Dispositif selon la revendication 1 dans lequel l'actionneur a un mouvement d'actionnement en va-et-vient le long de l'axe central.

4. Dispositif selon la revendication 1 dans lequel l'actionneur distribue de 0,02 à 0,05 g de poudre par actionnement.

5. Dispositif selon la revendication 1 dans lequel la pointe comprend une rampe de déviation de poudre (22) qui envoie la quasi-totalité de la poudre qui passe à l'écart de l'axe central.

6. Dispositif selon la revendication 5 dans lequel la rampe de la pointe est concave par rapport à un axe perpendiculaire à la direction principale d'écoulement de la poudre de façon à inciter la poudre qui passe à rester en contact avec la rampe pendant que la poudre est déviée et à inciter la poudre qui passe à ne pas rebondir de la rampe.

7. Dispositif selon la revendication 1 dans lequel la goulotte a une extrémité distale et des parois latérales qui convergent au fur et à mesure qu'elles s'approchent de l'extrémité distale de la goulotte, permettant ainsi de concentrer la poudre qui passe.

8. Dispositif selon la revendication 7 dans lequel la goulotte a un diamètre, mesuré comme une corde entre les parties supérieures des parois latérales de la goulotte, de 5 à 13 mm à proximité de la pointe et de 3 à 9 mm à proximité de l'extrémité distale de la goulotte.

9. Dispositif selon la revendication 1 comprenant en outre un embout (34) doté de parties formant ailettes légèrement arrondies et aplaties (38a, 38b) qui font saillie latéralement à l'écart de l'extrémité distale de la goulotte et aident à l'étalement de la poudre.

10. Dispositif selon la revendication 9 dans lequel l'embout est dépourvu d'arêtes tranchantes susceptibles de provoquer un traumatisme des tissus.

11. Dispositif selon la revendication 9 dans lequel l'embout présente une partie arrière en coupole qui aide à la rétractation des tissus et au ratissage et à l'étalement de la poudre distribuée sur le fond et de haut en bas des parois latérales d'un champ opératoire.

12. Dispositif selon la revendication 9 dans lequel l'embout est suffisamment rigide pour permettre l'étalement et le tassement de la poudre distribuée sans déviation notable de l'embout.

13. Dispositif selon la revendication 9 dans lequel l'embout peut être retiré du dispositif par un utilisateur et peut être changé ou remplacé par des embouts (46, 48) ayant des tailles différentes, des formes différentes ou des caractéristiques spécifiques du point de vue anatomique qui permettent de mieux adapter l'embout à un champ opératoire.

14. Dispositif selon la revendication 9 dans lequel l'embout s'étend en formant un angle de 4 à 60 degrés par rapport à l'axe central et a une longueur de 10 à 30 mm.

15. Dispositif selon la revendication 1 dans lequel le dispositif se présente dans un emballage stérilisé étanche.
